# EUROPEAN PATENT SPECIFICATION

(11) **EP 3 139 939 B1**
(45) Date of publication and mention of the grant of the patent: **06.09.2023**
(21) Application number: 15788711.8
(22) Date of filing: 07.05.2015
(51) Int. Cl.: A61K 35/66, A61K 35/74, A61K 35/76, A61P 17/00

(54) **COMPOSITIONS AND METHODS FOR TREATING SKIN AND MUCOUS MEMBRANE DISEASES**
ZUSAMMENSETZUNGEN UND VERFAHREN ZUR BEHANDLUNG VON HAUT- UND SCHLEIMHAUTMEMBRANERKRANKUNGEN
COMPOSITIONS ET MÉTHODES DE TRAITEMENT DE MALADIES DE LA PEAU ET DES MUQUEUSES

(30) Priority: 07.05.2014 US 201461989818 P
(43) Date of publication of application: 15.03.2017
(73) Proprietor: The Regents of the University of California, Oakland, CA 94607 (US); The United States Government as represented by the Department of Veterans Affairs, Washington DC 20420 (US)
(72) Inventor: KIM, Jenny, J., Los Angeles, CA 90095-3075 (US); CHAMPER, Jackson, Riverside, CA 92507 (US); YU, Yang, Arcadia, CA 91006 (US)
(74) Representative: Müller-Boré & Partner Patentanwälte PartG mbB
(86) International application number: PCT/US2015/029698
(87) International publication number: WO 2015/171899

(56) References cited:
- EP-A1- 0 914 778
- WO-A1-2013/142378
- US-A1- 2009 035 329
- US-B1- 6 726 913
- ELISABETH NAGY ET AL: "MALDI-TOF MS fingerprinting facilitates rapid discrimination of phylotypes I, II and III of Propionibacterium acnes", ANAEROBE, vol. 20, 1 April 2013 (2013-04-01), pages 20-26, XP55423428, GB ISSN: 1075-9964, DOI: 10.1016/j.anaerobe.2013.01.007
- MCDOWELL, A. ET AL.: 'The opportunistic pathogen Propionibacterium acnes: insights into typing, human disease, clonal diversification and CAMP factor evolution' PLOS ONE vol. 8, no. 9, 2013, pages 1 - 22, XP055237320
- FITZ-GIBBON, S. ET AL.: 'Propionibacterium acnes strain populations in the human skin microbiome associated with acne' JOURNAL OF INVESTIGATIVE DERMATOLOGY vol. 133, 2013, pages 2152 - 2160, XP055166702

## Description

### GOVERNMENT SUPPORT

This invention was made with Government support under AR053542, awarded by the National Institutes of Health, and by the U.S. Department of Veterans Affairs. The Government has certain rights in the invention.

### PRIORITY CLAIM

This patent application claims priority to U.S. Provisional Patent Application No. 61/989,818, filed on May 7, 2014.

### BACKGROUND

Acne affects up to 85% of adolescents and causes significant morbidity. Topical therapy is the first line treatment for mild acne and can be used as adjunctive therapy for moderate to severe acne. However, undesirable side effects such as dryness, redness, and irritation from topical therapies such as tretinoin derivatives and benzoyl peroxide limit patient compliance.

Acne pathogenesis is mediated by several factors including hyperproliferation and abnormal differentiation of keratinocytes, impaction of follicles forming a keratinaceous plug, increased androgens and sebum production, *Propionibacterium acnes* overgrowth, and the production of proinflammatory mediators.

It is known that antimicrobials such as antibiotics can be bactericidal or bacteriostatic, limiting and reducing the quantity of bacteria in our bodies. Retinoids also reduce the amount of bacteria in pilosebacous units and microcomedones. Light based therapies work via photothermal heating, photochemical inactivation of bacteria, and various photoimmunological reactions to improve clinical skin outcomes.

However, despite many commercially available therapies, acne remains a therapeutically challenging condition, with many patients being unresponsive to several attempted therapies, making treatment unpredictable and elusive in many cases.

WO 2013/142378 A1 describes fast diagnosis and personalized treatments for acne. Elisabeth Nagy et al. (Anaerobe, vol. 20, p. 20 -26) report that MALDI-TOF MS fingerprinting facilitates rapid discrimination of phylotypes I, II and III of Propionibacterium acnes.

### SUMMARY

In some aspects, the invention relates to a topical formulation for use in a method of inhibiting growth of acne-associated *Propionebacterium acnes* in a subject in need thereof, the topical formulation comprising between 1×10³ and 1×10¹⁰ Phylotype III *Propionibacterium acnes* cells per milliliter, the method comprising:
applying to the skin of the subject the topical formulation, and
prior to applying the topical formulation, administering to the subject a composition comprising an antimicrobial agent selected from the group consisting of clindamycin, erythromycin, sulfacetamide sodium/sulfur, silver sulfadiazine, neomycin, polymyxin B, bacitracin, mupirocin, and retapamulin.

In some aspects, the invention relates to a kit for use in a method of inhibiting growth of acne-associated *Propionebacterium acnes,* comprising:
a composition comprising an antimicrobial agent selected from the group consisting of clindamycin, erythromycin, sulfacetamide sodium/sulfur, silver sulfadiazine, neomycin, polymyxin B, bacitracin, mupirocin, retapamulin; and
a topical formulation as defined above.

### BRIEF DESCRIPTION OF THE FIGURES

**Figure 1****.** Demonstration of RT6 probiotic use in conjunction with antibiotic treatment. Figure 1 shows the amount of each type of bacterial strain as determined by CFU assay.
**Figure 2****.** Demonstration of phylotype III probiotic use in conjunction with antibiotic treatment. Figure shows amount of each type of bacterial strain as determined by CFU assay.
**Figure 3**. IL-10 secretion by PBMCs stimulated by different strains of *P. acnes.* IL-10 secretion was measured by ELISA. Representative of three experiments. Statistics show t-test comparison to RT6. *** p<0.001.
**Figure 4****.** IL-10 secretion by PBMCs stimulated by different strains of *P. acnes.* IL-10 secretion was measured by ELISA. Representative of three experiments. Statistics show t-test comparison to Phylotype III. *** p<0.001.
**Figure 5**. II,-17 secretion by PBMCs stimulated by different strains of *P. acnes.* II,-17 secretion was measured by ELISA. Representative of three experiments. Statistics show t-test comparison to RT6. ** p<0.01, ** p<0.001.

### DETAILED DESCRIPTION

### Overview

Oral probiotics have been studied for treating and/or preventing many diseases, including those of the gastrointestinal tract and even inflammatory skin disorders such as acne vulgaris. Topical probiotics, however, have not been well studied.

Studies have demonstrated that the addition of certain bacterial strains may outcompete and inhibit the growth of skin-related pathogenic bacteria such as *Propionibacterium acnes,* the bacteria most associated with acne, or *Staphylococcus aureus,* which causes many infections of the skin such as impetigo, cellulitis, and others. Specifically, *in vitro* experiments showed that *Lactobacillus casei, Lactobacillus acidophilus, Lactobacillus plantarum, Lactobacillus gasseri,* and *Lactococcus lactis* inhibit growth of *Propionibacterium acnes* on agar plates (Al-Ghazzewi & Tester, Int J Cosmet Sci 32:139-42 (2010)). Another study showed that the presence of *Lactobacillus reuteri* and *Lactobacillus rhamnosus* reduced death of keratinocytes in vitro caused by *Staphylococcus aureus* (Prince et al., Appl Environ Microbiol, 78:5119-26 (2012)). *Propionibacterium acnes* was found to slow growth of *Staphylococcus aureus in vitro* and on mouse skin wounds (Shu et al., PLoS One, 8:55380 (2013)).

The presently-disclosed invention is rooted in the discovery that *Propionibacterium acnes,* the species of bacteria that causes acne, may be used to treat acne by administering non-pathogenic or low-pathogenic strains of *Propionibacterium acnes* to the skin or a mucous membrane to either (1) compete with pathogenic strains, or (2) to repopulate the skin or a mucous membrane following treatment with an antimicrobial agent.

In some aspects, the invention relates to topical formulations for use in a method of inhibiting growth of acne-associated Propionebacterium acnes in a subject in need thereof, the topical formulation comprising between 1×10³ and 1×10¹⁰ *Propionibacterium acnes* Phylotype III cells per mililiter.
The method comprises two steps. The first step consists of administering a composition comprising an antimicrobial agent selected from the group consisting of clindamycin, erythromycin, sulfacetamide sodium/sulfur, silver sulfadiazine, neomycin, polymyxin B, bacitracin, mupirocin, and retapamulin. The second step consists of administering a topical formulation comprising between 1×10³ and 1×10¹⁰ Phylotype III Propionibacterium acnes cells per milliliter. Thus, bacteria from the topical formulation repopulate the skin or a mucous membrane following antimicrobial therapy

In some aspects, the invention relates to a kit for use in a method of inhibiting growth of acne-associated Propionebacterium acne, comprising an antimicrobial agent selected from the group consisting of clindamycin, erythromycin, sulfacetamide sodium/sulfur, silver sulfadiazine, neomycin, polymyxin B, bacitracin, mupirocin, retapamulin and a topical formulation as defined above.

### Definitions

As used herein the specification, "a" or "an" may mean one or more. As used herein in the claim(s), when used in conjunction with the word "comprising", the words "a" or "an" may mean one or more than one. As used herein "another" may mean at least a second or more.

The term "low-pathogenic" refers to strains of bacteria, fungus, and viruses that do not produce clinical symptoms in a majority of subjects, including naturally-occurring strains and genetically-modified strains.

The term "non-pathogenic" refers to strains of bacteria, fungus, and viruses that do not produce clinical symptoms in a subject, including naturally-occurring strains and genetically-modified strains. Phylotype III *Propionibacterium acnes* is an example of a non-pathogenic bacteria.

The term "preventing" is art-recognized, and when used in relation to a condition, such as a local recurrence, is well understood in the art, and includes administration of a composition which reduces the frequency of, or delays the onset of, symptoms of a medical condition in a subject relative to a subject which does not receive the composition. Thus, prevention of acne includes, for example, reducing the number of detectable acne lesions in a population of patients receiving a prophylactic treatment relative to an untreated control population, and/or delaying the appearance of detectable lesions in a treated population versus an untreated control population, e.g., by a statistically and/or clinically significant amount.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic (i.e., it protects the host against developing the unwanted condition), whereas if it is administered after manifestation of the unwanted condition, the treatment is therapeutic (i.e., it is intended to diminish, ameliorate, or stabilize the existing unwanted condition or side effects thereof).

The term "ribotype" refers to strains of *P. acnes.* The ribotyped strains were characterized as in Fitz-Gibbon et al. (J. Investigative Dermatology 133:2152-60 (2013)).

The term "phylotype" refers to strains of *P. acnes.* The phylotyped strains were characterized as in McDowell et al. (PLoS ONE 8(9): e70897 (2013)).

The term "subject" refers to a mammal, including, but not limited to, a human or non-human mammal, such as a bovine, equine, canine, ovine, or feline.

A "therapeutically effective amount" of a compound with respect to the subject method of treatment refers to an amount of the compound(s) in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

As used herein, the term "treating" or "treatment" includes reversing, reducing, or arresting the symptoms, clinical signs, and underlying pathology of a condition in a manner to improve or stabilize a subject's condition.

### Antimicrobial therapy

It is known that antimicrobials such as antibiotics can be bactericidal or bacteriostatic, limiting and reducing the quantity of bacteria in our bodies. Retinoids also reduce the amount of bacteria in pilosebacous units and microcomedones. Light based therapies work via photothermal heating, photochemical inactivation of bacteria, and various photoimmunological reactions to improve clinical skin outcomes. In general, the above therapies directly act to reduce the amount of pathogenic bacteria in a patient. Thus, the invention proposes that any such therapy that achieves the same goal of reducing the number of pathogenic organisms, when used in combination with subsequent topical probiotic treatment, would lead to replacement of the pathogenic microflora involved in the diseased state with natural microflora enriched in healthy skin or mucous membranes, or less pathogenic species occupying the same ecological niche as the type causing a disease state.

According to the present invention, the antimicrobial agent is selected from the group consisting of clindamycin, erythromycin, sulfacetamide sodium/sulfur, silver sulfadiazine, neomycin, polymyxin B, bacitracin, mupirocin, and retapamulin

### Probiotic composition

According to the present invention, the topical formulation for use in a method of inhibiting growth of acne-associated Propionebacterium acnes in a subject in need thereof comprises between 1×10³ and 1×10¹⁰ Phylotype III Propionibacterium acnes cells per milliliter.

In some embodiments, the topical formulation comprises 1×10³, 5×10³, 1×10⁴, 5×10⁴, 1×10⁵, 5×10⁵, 1×10⁶, 5×10⁶, 1×10⁷, 5×10⁷, 1×10⁸, 5×10⁸, 1×10⁹, 5×10⁹, or 1×10¹⁰ bacteria cells per milliliter.

In some embodiments, the topical formulation comprises a non-pathogenic and/or low-pathogenic species of Proteobacteria, Actinobacteria, or Firmicutes. In preferred embodiments, the topical formulation comprises a non-pathogenic and/or low-pathogenic species of *Corynebacterium, Bacillus Bifidobacterium, Janthinobacterium, Lactobacillus, Pseudomonas, Propionibacterium,* or *Staphylococcus.* In other preferred embodiments, the topical formulation comprises a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* or *Pseudomonas lindbergii.*

In some preferred embodiments, the topical formulation comprises between approximately 1×10⁴ and 1×10⁸ *Propionibacterium acnes* cells per milliliter. In more preferred embodiments, the topical formulation comprises between approximately 1×10⁵ and 5×10⁷ *Propionibacterium acnes* cells per milliliter.

In some embodiments, the topical formulation comprises an effective amount of a topical retinoid. In some embodiments, the retinoid is retinol, adapalene, tretinoin, or tazarotene.

In some embodiments, the topical formulation comprises a topical antifungal agent. In some embodiments, the topical antifungal agent is ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil.

In some embodiments, the topical formulation comprises an effective amount of a phage. In some embodiments, the phage is P1, P9, P14, P100A, P100D, P100.1, P101A, P104A, P105, or ATCC_C.

In some embodiments, the topical formulation is an emulsion, cream, lotion, gel, oil, ointment, aerosol spray, or semi-solid formulation. In some embodiments, the topical formulation comprises a carrier, wherein said carrier is selected from the group consisting of trehalose, malto-dextrin, rice flour, micro-crystalline cellulose, magnesium stearate, inositol, fructo-oligosaccharide, gluco-oligosaccharide, dextrose, sucrose, talc, water, physiological salt solution, urea, methanol, ethanol, propanol, butanol, ethylene glycol, propylene glycol, white pertrolatum, isopropyl myristate, lanolin, lanolin alcohol, mineral oil, lavender oil, nasturtium extract oil, sorbitan mono-oleate, cetylstearyl alcohol, hydroxypropyl cellulose, detergent, sucrose stearate, sucrose cocoate, sucrose distearate, 2-ethyl-1,3-hexanediol, polyoxypropylene-15-stearyl ether, glycerol stearate, glycerin, synthetic spermaceti, cetyl alcohol, butylparaben, propylparaben, and methylparaben.

In some embodiments, the invention relates to a kit for use in a method of inhibiting growth of acne-associated Propionebacterium acnes, wherein the kit comprises (1) a composition comprising an antimicrobial agent selected from the group consisting of clindamycin, erythromycin, sulfacetamide sodium/sulfur, silver sulfadiazine, neomycin, polymyxin B, bacitracin, mupirocin, retapamulin and (2) a topical formulation according to the present invention. In some embodiments, the composition is adapted for topical administration.

### EXEMPLIFICATION

### Reference Example 1: Formulation containing P. acnes ribotype 6

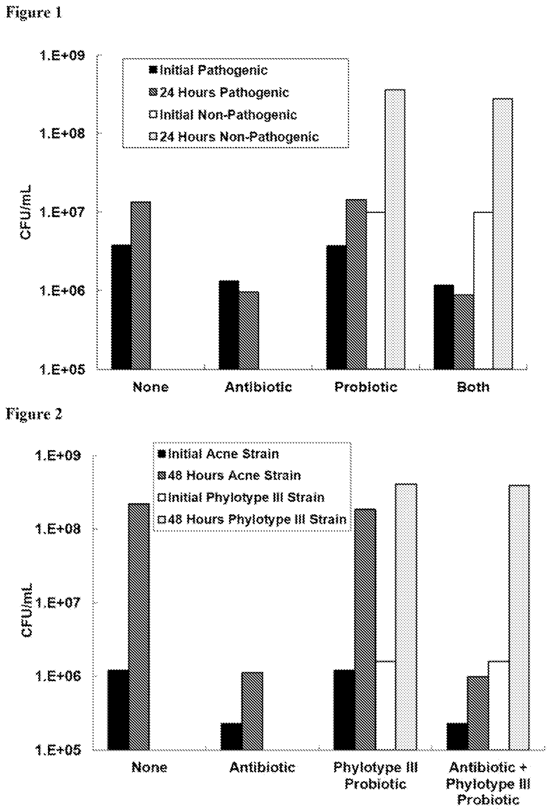

To demonstrate the efficacy of combining the unique formulation of non-pathogenic bacteria in combination with antimicrobial treatment, growth experiments were conducted with *P. acnes* of non-pathogenic ribotype 6 and of acne-associated, pathogenic ribotypes 5 and 8 (Figure 1, Table I). All conditions initially contained a low concentration of acne-associated, pathogenic strains growing in media. Antibiotic treated condition contained sufficient antibiotics to eliminate 90% of bacteria, but as in disease, remaining bacteria were able to grow back. In the probiotic condition, a similar amount of non-pathogenic bacteria were added initially to the media containing the pathogenic strain. The non-pathogenic strains grew faster than the pathogenic strain, and eventually the percentage of pathogenic bacteria was greatly reduced. The amount of pathogenic bacteria was further reduced in the combined treatment, in which the non-pathogenic bacteria were added after antibiotic treatment of the pathogenic strains.

**Table I**

| Demonstration of RT6 topical probiotic use for acne after antibiotic treatment. Table shows % of each type of bacterial strain as determined by CFU assay. | | | | | |
|---|---|---|---|---|---|
| | **Treatment** | **Initial Acne %** | **24h Acne %** | **Initial RT6 %** | **24h RT6 %** |
| | **None** | 100 | 100 | 0 | 0 |
| | **Antibiotic** | 100 | 100 | 0 | 0 |
| | **RT6 Probiotic** | 27.0 | 3.9 | 73.0 | 96.1 |
| | **Antibiotic + RT6** | 10.5 | 0.3 | 89.5 | 99.7 |

### Example 2: Formulation containing Phylotype III P. acnes

An additional experiment using healthy skin associated Phylotype III *P. acnes* yielded similar results (Figure 2, Table II). The combination of antibiotic treatment followed by probiotic treatment yielded a synergistic improvement because the percentage of pathogenic bacteria in this combination treatment (Tables I and II) was significantly decreased compared to an additive effect of both individual treatments. Lower percentages of pathogenic strains in the total composition of bacterial flora on the skin indicate a reduced likelihood of developing acne in a patient. At 48hrs, the antibiotic treatment alone does not change the percentage of pathogenic strains in the total composition, while the probiotic treatment alone significantly reduces the percentage of pathogenic strains. However, the combination treatment at 48hrs synergistically reduced the percentage of pathogenic bacteria to only 0.3%, a reduction of 1-2 orders of magnitude compared to probiotic alone. Thus, a properly timed combination of these treatments in the clinic (antibiotic followed by probiotic at an appropriate interval) may yield synergistic improvements in the disease state compared to either treatment alone, or compared to an improperly implemented combination of these treatments (both treatments at the same time or probiotic treatment before antibiotic treatment).

**Table II**

| Demonstration of phylotype III topical probiotic use for acne after antibiotic treatment. Table shows % of each type of bacterial strain as determined by CFU assay. | | | | |
|---|---|---|---|---|
| **Treatment** | **Initial Acne %** | **48h Acne %** | **Initial Type III %** | **48h Type III %** |
| **None** | 100 | 100 | 0 | 0 |
| **Antibiotic** | 100 | 100 | 0 | 0 |
| **Phylotype III Probiotic** | 42.9 | 31.3 | 57.1 | 68.7 |
| **Antibiotic + Phylotype III** | 12.6 | 0.3 | 87.4 | 997 |

### Reference Example 3: Analysis of P. acnes strains

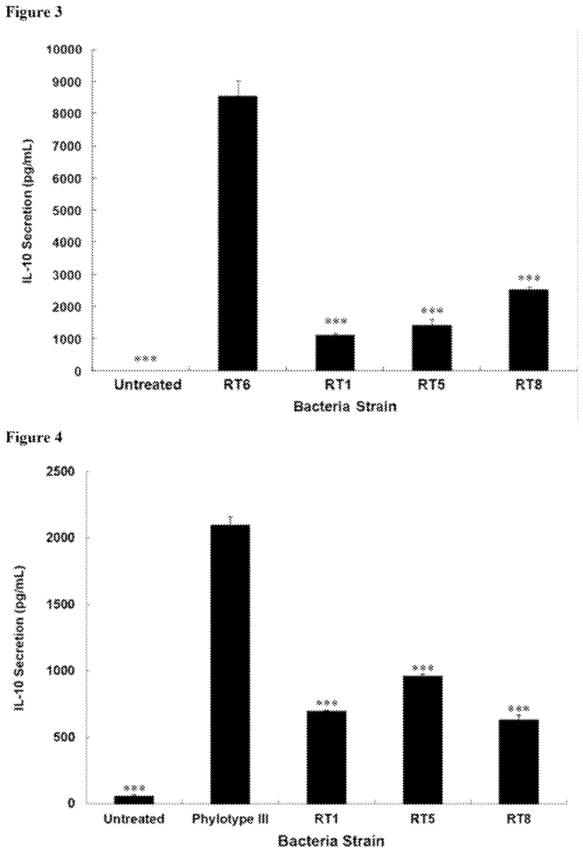

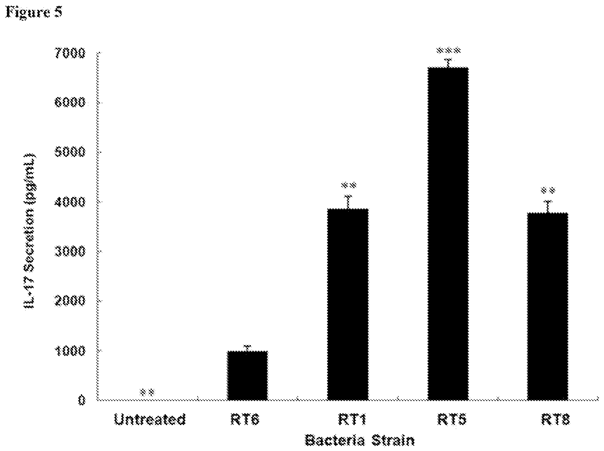

To demonstrate reduced virulence of the strains associated with only healthy skin (ribotype 6 and phylotype III) as compared to acne-associated strains (ribotypes 5 and 8) and strains found in both healthy and acne-affected skin (ribotype 1), peripheral blood mononuclear cells (PBMCs) were stimulated with live *P. acnes.* The ribotype 6 (Figure 3) and phylotype III (Figure 4) strains induced secretion of significantly higher levels of antiinflammatory IL-10 than ribotypes 5, 8, and 1. IL-17 is an inflammatory cytokine thought to play a role in acne pathogenesis. The ribotype 6 strain induced lower IL-17 secretion, while other strains showed significantly higher induction of IL-17 secretion (Figure 5). This indicates that an increase in healthy skin-associated strains compared to acne-associated strains may reduce inflammation in acne, leading to an improvement in the disease state.

## Claims

1. A topical formulation for use in a method of inhibiting growth of acne-associated *Propionibacterium acnes* in a subject in need thereof, the topical formulation comprising between 1×10³ and 1×10¹⁰ Phylotype III *Propionibacterium acnes* cells per milliliter, the method comprising:
applying to the skin of the subject the topical formulation, and
prior to applying the topical formulation, administering to the subject a composition comprising an antimicrobial agent selected from the group consisting of clindamycin, erythromycin, sulfacetamide sodium/sulfur, silver sulfadiazine, neomycin, polymyxin B, bacitracin, mupirocin, and retapamulin.

2. The topical formulation of claim 1, further comprising a non-pathogenic and/or low-pathogenic species of Proteobacteria, Actinobacteria, or Firmicutes, wherein low-pathogenic species are species that do not produce clinical symptoms in a majority of subjects.

3. The topical formulation of claim 1 or 2, further comprising a non-pathogenic and/or low-pathogenic species of *Corynebacterium, Bacillus Bifidobacterium, Janthinobacterium, Lactobacillus, Pseudomonas, Propionibacterium,* or *Staphylococcus,* wherein low-pathogenic species are species that do not produce clinical symptoms in a majority of subjects.

4. The topical formulation of any one of claims 1-3, further comprising a non-pathogenic and/or low-pathogenic *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus,* or *Pseudomonas lindbergii,* wherein low-pathogenic species are species that do not produce clinical symptoms in a majority of subjects.

5. The topical formulation of any one of the preceding claims, further comprising an effective amount of a topical retinoid, such as retinol, adapalene, tretinoin, or tazarotene.

6. The topical formulation of any one of the preceding claims, further comprising a topical antifungal agent, such as ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil.

7. The topical formulation of any one of the preceding claims, further comprising an effective amount of a phage, such as P1, P9, P14, P100A, P100D, P100.1, P101A, P104A, P105, or ATCC_C.

8. The topical formulation of any one of the preceding claims, wherein the formulation is an emulsion, cream, lotion, gel, oil, ointment, aerosol spray, or semi-solid formulation.

9. The topical formulation of any one of the preceding claims, further comprising a carrier, wherein said carrier is selected from the group consisting of trehalose, maltodextrin, rice flour, micro-crystalline cellulose, magnesium stearate, inositol, fructo-oligosaccharide, gluco-oligosaccharide, dextrose, sucrose, talc, water, physiological salt solution, urea, methanol, ethanol, propanol, butanol, ethylene glycol, propylene glycol, white pertrolatum, isopropyl myristate, lanolin, lanolin alcohol, mineral oil, lavender oil, nasturtium extract oil, sorbitan mono-oleate, cetylstearyl alcohol, hydroxypropyl cellulose, detergent, sucrose stearate, sucrose cocoate, sucrose distearate, 2-ethyl-1,3-hexanediol, polyoxypropylene-15-stearyl ether, glycerol stearate, glycerin, synthetic spermaceti, cetyl alcohol, butylparaben, propylparaben, and methylparaben.

10. A kit for use in a method of inhibiting growth of acne-associated *Propionibacterium acnes,* comprising:
a composition comprising an antimicrobial agent selected from the group consisting of clindamycin, erythromycin, sulfacetamide sodium/sulfur, silver sulfadiazine, neomycin, polymyxin B, bacitracin, mupirocin, retapamulin; and
a topical formulation of any one of claims 1-9.

11. The kit of claim 10, wherein the composition is adapted for topical administration.

## Patentansprüche

1. Topische Formulierung zur Verwendung in einem Verfahren zur Hemmung des Wachstums von mit Akne assoziiertem *Propionebacterium acnes* in einem Individuum, das dessen bedarf, wobei die topische Formulierung zwischen 1×10³ und 1×10¹⁰ Zellen des Phylotyps III von *Propionibacterium acnes* pro Milliliter umfasst, wobei das Verfahren umfasst:
Auftragen der topischen Formulierung auf die Haut des Individuums, und
vor dem Auftragen der topischen Formulierung dem Individuum eine Zusammensetzung verabreichen, die ein antimikrobielles Mittel, ausgewählt aus der Gruppe bestehend aus Clindamycin, Erythromycin, Sulfacetamid-Natrium/Schwefel, Silbersulfadiazin, Neomycin, Polymyxin B, Bacitracin, Mupirocin und Retapamulin, enthält.

2. Topische Formulierung nach Anspruch 1, weiter umfassend eine nicht-pathogene und/oder niedrig-pathogene Spezies von Proteobakterien, Actinobakterien oder Firmicutes, wobei niedrig-pathogene Spezies Spezies sind, die bei der Mehrheit der Individuen keine klinischen Symptome hervorrufen.

3. Topische Formulierung nach Anspruch 1 oder 2, weiter umfassend eine nichtpathogene und/oder niedrig-pathogene Spezies von *Corynebacterium, Bacillus Bifidobacterium, Janthinobacterium, Lactobacillus, Pseudomonas, Propionibacterium* oder *Staphylococcus,* wobei niedrig-pathogene Spezies Spezies sind, die bei der Mehrheit der Individuen keine klinischen Symptome hervorrufen.

4. Topische Formulierung nach einem der Ansprüche 1 bis 3, weiter umfassend einen nicht-pathogenen und/oder niedrig-pathogenen *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus* oder *Pseudomonas lindbergii,* wobei niedrig-pathogene Spezies Spezies sind, die bei der Mehrheit der Individuen keine klinischen Symptome hervorrufen.

5. Topische Formulierung nach einem der vorhergehenden Ansprüche, weiter umfassend eine wirksame Menge eines topischen Retinoids, wie Retinol, Adapalen, Tretinoin oder Tazaroten.

6. Topische Formulierung nach einem der vorhergehenden Ansprüche, weiter umfassend ein topisches Antimykotikum, wie Ketoconazol, Miconazol, Fluconazol, Clotrimazol, Undecylensäure, Sertaconazol, Terbinafin, Butenafin, Clioquinol, Haloprogin, Nystatin, Naftifin, Tolnaftat, Ciclopirox, Amphotericin B oder Teebaumöl.

7. Topische Formulierung nach einem der vorhergehenden Ansprüche, weiter umfassend eine wirksame Menge eines Phagen, wie P1, P9, P14, P100A, P100D, P100.1, P101A, P104A, P105 oderATCC_C.

8. Topische Formulierung nach einem der vorhergehenden Ansprüche, wobei die Formulierung eine Emulsion, Creme, Lotion, Gel, Öl, Salbe, Aerosolspray oder halbfeste Formulierung ist.

9. Topische Formulierung nach einem der vorhergehenden Ansprüche, weiter umfassend einen Träger, wobei der Träger ausgewählt ist aus der Gruppe bestehend aus Trehalose, Maltodextrin, Reismehl, mikrokristalliner Cellulose, Magnesiumstearat, Inosit, Fructo-Oligosaccharid, Gluco-Oligosaccharid, Dextrose, Saccharose, Talk, Wasser, physiologischer Salzlösung, Harnstoff, Methanol, Ethanol, Propanol, Butanol, Ethylenglykol, Propylenglykol, weißes Pertrolatum, Isopropylmyristat, Lanolin, Lanolinalkohol, Mineralöl, Lavendelöl, Kapuzinerkresseöl, Sorbitanmonooleat, Cetylstearylalkohol, Hydroxypropylcellulose, Detergens, Saccharosestearat, Saccharosecocoat, Saccharosedistearat, 2-Ethyl-1,3-Hexandiol, Polyoxypropylen-15-stearylether, Glycerinstearat, Glycerin, synthetisches Spermaceti, Cetylalkohol, Butylparaben, Propylparaben und Methylparaben.

10. Kit zur Verwendung in einem Verfahren zur Hemmung des Wachstums von mit Akne-assoziiertem *Propionebacterium acnes,* umfassend:
eine Zusammensetzung, umfassend ein antimikrobielles Mittel ausgewählt aus der Gruppe bestehend aus Clindamycin, Erythromycin, Sulfacetamid-Natrium/Schwefel, Silbersulfadiazin, Neomycin, Polymyxin B, Bacitracin, Mupirocin, Retapamulin; und
eine topische Formulierung nach einem der Ansprüche 1-9.

11. Kit nach Anspruch 10, wobei die Zusammensetzung für die topische Verabreichung angepasst ist.

## Revendications

1. Formulation topique destinée à être utilisée dans un procédé d'inhibition de la croissance de *Propionibacterium acnes* associée à l'acné chez un sujet en ayant besoin, la formulation topique comprenant entre 1×10³ et 1×10¹⁰ cellules de *Propionibacterium acnes* de phylotype III par millilitre, le procédé comprenant :
appliquer à la peau du sujet la formulation topique, et
avant d'appliquer la formulation topique, administrer au sujet une composition comprenant un agent antimicrobien sélectionné parmi le groupe constitué de la clindamycine, l'érythromycine, le sulfacétamide de sodium/soufre, la sulfadiazine argentique, la néomycine, la polymyxine B, la bacitracine, la mupirocine et la rétapamuline.

2. Formulation topique selon la revendication 1, comprenant en outre une espèce non pathogène et/ou faiblement pathogène de Proteobacteria, Actinobacteria ou Firmicutes, dans laquelle les espèces faiblement pathogènes sont des espèces qui ne produisent pas de symptômes cliniques chez une majorité de sujets.

3. Formulation topique selon la revendication 1 ou 2, comprenant en outre une espèce non pathogène et/ou faiblement pathogène de *Corynebacterium, Bacillus Bifidobacterium, Janthinobacterium, Lactobacillus, Pseudomonas, Propionibacterium* ou *Staphylococcus,* dans laquelle les espèces faiblement pathogènes sont des espèces qui ne produisent pas de symptômes cliniques chez une majorité de sujets.

4. Formulation topique selon l'une quelconque des revendications 1 à 3, comprenant en outre *Bacillus coagulans, Bacillus laterosporus, Bacillus megaterium, Bacillus polymyxa, Bacillus licheniformis, Bacillus pumilus, Bacillus stearothermophilus, Bacillus subtilis, Bacillus uniflagellatus, Bifidobacterium adolescentis, Bifidobacterium animalis, Bifidobacterium bifidum, Bifidobacterium breve, Bifidobacterium infantis, Bifidobacterium lactis, Bifidobacterium longum, Bifidobacterium pseudocatenulatur, Janthinobacterium lividum, Lactobacillus acidophilus, Lactobacillus casei, Lactobacillus fermentum, Lactobacillus gasseri, Lactobacillus johnsonii, Lactobacillus lactis, Lactobacillus plantarum, Lactobacillus paracasei, Lactobacillus reuteri, Lactobacillus rhamnosus* ou *Pseudomonas lindbergii* non pathogène et/ou faiblement pathogène, dans laquelle les espèces faiblement pathogènes sont des espèces qui ne produisent pas de symptômes cliniques chez une majorité de sujets.

5. Formulation topique selon l'une quelconque des revendications précédentes, comprenant en outre une quantité efficace d'un rétinoïde topique, tel que le rétinol, l'adapalène, la trétinoïne ou le tazarotène.

6. Formulation topique selon l'une quelconque des revendications précédentes, comprenant en outre un agent antifongique topique, tel que le kétoconazole, le miconazole, le fluconazole, le clotrimazole, l'acide undécylénique, le sertaconazole, la terbinafine, la buténafine, le clioquinol, l'haloprogin, la nystatine, la naftitine, le tolnaftate, le ciclopirox, l'amphotéricine B ou l'huile d'arbre à thé.

7. Formulation topique selon l'une quelconque des revendications précédentes, comprenant en outre une quantité efficace d'un phage, tel que P1, P9, P14, P100A, P100D, P100.1, P101A, P104A, P105 ou ATCC_C.

8. Formulation topique selon l'une quelconque des revendications précédentes, dans laquelle la formulation est une émulsion, une crème, une lotion, un gel, une huile, une pommade, un spray aérosol ou une formulation semi-solide.

9. Formulation topique selon l'une quelconque des revendications précédentes, comprenant en outre un support, dans laquelle ledit support est sélectionné parmi le groupe constitué du tréhalose, de la maltodextrine, de la farine de riz, de la cellulose microcristalline, du stéarate de magnésium, de l'inositol, d'un fructo-oligosaccharide, d'un gluco-oligosaccharide, du dextrose, du saccharose, du talc, de l'eau, d'une solution de sel physiologique, de l'urée, du méthanol, de l'éthanol, du propanol, du butanol, de l'éthylèneglycol, du propylèneglycol, de pétrolatum blanc, du myristate d'isopropyle, de la lanoline, de l'alcool de lanoline, d'une huile minérale, de l'huile de lavande, de l'huile d'extrait de nasturtium, du mono-oléate de sorbitan, de l'alcool cétylstéarylique, de l'hydroxypropylcellulose, d'un détergent, du stéarate de saccharose, du cocoate de saccharose, du distéarate de saccharose, du 2-éthyl-1,3-hexanediol, de l'éther polyoxypropylène-15-stéarylique, du stéarate de glycérol, de la glycérine, de spermacéti synthétique, de l'alcool cétylique, du butylparabène, du propylparabène et du méthylparabène.

10. Kit destiné à être utilisé dans un procédé d'inhibition de la croissance de *Propionibacterium acnes* associée à l'acné, comprenant :
une composition comprenant un agent antimicrobien sélectionné parmi le groupe constitué de la clindamycine, l'érythromycine, le sulfacétamide de sodium/soufre, la sulfadiazine argentique, la néomycine, la polymyxine B, la bacitracine, la mupirocine, la rétapamuline ; et
une formulation topique selon l'une quelconque des revendications 1 à 9.

11. Kit selon la revendication 10, dans lequel la composition est adaptée à l'administration par voie topique.
